# EUROPEAN PATENT APPLICATION

(11) **EP 2 380 622 A1**
(43) Date of publication of application: **26.10.2011**
(21) Application number: 11174013.0
(22) Date of filing: 29.04.2005
(51) Int. Cl.: A61M 31/00

(54) **Method for ocular treatment**

(30) Priority: 29.04.2004 US 566776 P
(62) Divisional of application: 05742971.4
(71) Applicant: iScience Interventional Corporation, Menlo Park, CA 94025 (US)
(72) Inventor: Yamamoto, Ronald, San Francisco, CA California 94117 (US); Hee, Michael, Burlingame, CA California 94010 (US); Nash, Michael, Los Altos, CA California 94022 (US); McKenzie, John, San Carlos, CA California 94070 (US); Kupiecki, David, J., San Francisco, CA California 94117 (US); Conston, Stanley R., San Carlos, CA California 94070 (US)
(74) Representative: Neilson, Martin Mark

(57) **Abstract**

The invention provides tools, materials and related methods to surgically access the suprachoroidal space of an eye for the purpose of performing minimally invasive surgery or to deliver drugs to the eye. The invention provides a flexible microcannula device **(11, 13)** that may be placed into the suprachoroidal space **(12, 14)** through a small incision **(12A)** of the overlying tissues, maneuvered into the appropriate region of the space, and then activated to treat tissues adjacent to the distal tip of the device.

## Description

### PRIORITY FROM RELATED APPLICATION

Priority is hereby claimed from U.S. Provisional Application Serial No. 60/566,776, filed April 29, 2004, which is incorporated by reference herein in its entirety.

### BACKGROUND OF THE INVENTION

The eye is a complex organ with a variety of specialized tissues that provide the optical and neurological processes for vision. Accessing the eye for medical treatment is hindered by the small size and delicate nature of the tissues. Surgical access must not affect the optical clarity or alignment of the tissues in the visual axis to preserve vision. In addition, the eye is immunologically privileged, rendering it susceptible to severe infection, especially when the intraocular space is challenged by both pathogens and trauma.

Minimally invasive surgical methods to access and treat tissues of the eye are desired to minimize trauma and introduction of pathogens. Dissection of the eye during surgery may affect the optical alignment of tissues involved in vision and typically results in scarring which makes subsequent surgery more difficult. Minimally invasive surgical methods are advantageous in that they minimize potential alterations to the optical alignment of the tissues in the visual axis. Minimally invasive surgical methods may also allow for the use of small incisions, thereby limiting scarring and allowing subsequent surgical procedures to be performed.

Minimally invasive methods are routinely used in eye surgery to treat cataracts. Small incisions are made into the cornea and appropriately sized tools introduced and used under direct visualization through the cornea with a surgical microscope. The tools are used to remove the opacified natural lens and replace it with an intraocular lens implant. Minimally invasive methods are also used in retinal surgery, involving the introduction of tools into the posterior chamber of the eye through small incisions in the pars plana region of the sclera. Direct visualization through the cornea and visual axis with a surgical microscope allows the surgeon to manipulate tools to treat the retina and macula.

The present invention describes microsurgical tools and methods, which enable minimally invasive surgical access to the eye from within the suprachoroidal space. The suprachoroidal space is a virtual space between the sclera and choroid, due to the close apposition of the two tissues from the intraocular pressure of the eye. Although the suprachoroidal space is delicate in nature and is adjacent to numerous choroidal blood vessels, the present invention provides a flexible, catheter-like tool that may be safely placed in the suprachoroidal space and maneuvered anteriorly to the region near the cilliary body as well as posteriorly to the area of the retina and optic nerve. Such tools may be used to surgically treat the uveal scleral drainage pathway to increase aqueous outflow in the treatment of glaucoma, to surgically treat the macula and choroidal vasculature in the treatment of macular degeneration as well as to deliver drugs to the posterior tissues of the eye in the treatment of macular degeneration or optic nerve damage.

### SUMMARY OF THE INVENTION

The present invention provides a composite microcannula device with proximal and distal ends for access and advancement within the suprachoroidal space of the eye comprising, a flexible tubular sheath having an outer diameter of up to about 1000 microns and configured to fit within the suprachoroidal space of the eye; a proximal assembly configured for introduction and removal of materials and tools through the proximal end; and a signal-producing beacon at the distal end to locate the distal end within the eye, wherein the signal-producing beacon is detectable visually or by non-invasive imaging.

The signal-producing beacon may be configured to emit visible light at an intensity that is visible externally through interposing tissues or the beacon may comprise markers identifiable by non-invasive imaging, such as, ultrasound imaging, optical coherence tomography or ophthalmoscopy. The marker, for example may be an optical contrast marker. The beacon may provide illumination from the distal end at an angle of about 45 to about 135 degrees from the axis of the device to be coincident with the area of intended tissue treatment.

The tubular sheath is preferably curved in the range of 12 to 15 mm radius and may accommodate at least one additional signal-producing beacon detectable visually or by non-invasive imaging to aid in judging placement and location. Typically, the sheath comprises a lubricious outer coating and may have an atraumatic distal tip. The device preferably has a minimum length in the range of about 20 to about 30 mm to reach the posterior region of the eye from an anterior dissection into the suprachoroidal space.

The device may comprise an optical fiber for imaging tissues within or adjacent to the suprachoroidal space and an energy-emitting source for treating blood vessels within or adjacent to the suprachoroidal space. The source may be capable, for example, of emitting laser light, thermal energy, ultrasound, or electrical energy. Preferably the source is aligned with the location of the beacon to facilitate tissue targeting.

The device may further comprise an implant deliverable at the distal end. The implant may comprise a space-maintaining material or a drug.

The device may further comprise a sustained release drug formulation deliverable at the distal end.

In another embodiment, the device additionally comprises an inner member with a proximal end and a distal end, wherein the sheath and inner member are sized such that the inner member fits slidably within the sheath and the distal end of the inner member is adapted to provide tissue treatment to the eye through one or more openings in the distal end. The distal end of the inner member may be adapted for tissue dissection, cutting, ablation or removal. The inner member may be curved in the range of 12 to 15 mm radius and may comprise a multi-lumen tube and/or an optical fiber. The inner member may be made of steel, nickel titanium alloy or tungsten.

In another embodiment, a composite microcannula device is provided for implantation in the suprachoroidal space of an eye for delivery of fluids to the posterior region of the eye comprising, a flexible tubular sheath having proximal and distal ends with an outer diameter of up to about 1000 microns configured to fit within the suprachoroidal space of the eye; a self-sealing proximal fitting capable of receiving injections of fluids into the device, wherein the distal end of the sheath is adapted for release of fluids from the device into the eye.

The device may comprise a signal-producing beacon to locate the distal end within the suprachoroidal space during implantation wherein the signal-producing beacon is detectable visually or by non-invasive imaging. The device may be adapted for slow release of fluids, such as drugs, from the distal end.

In another embodiment, a method is provided for treating the suprachoroidal space of an eye comprising
a) inserting a flexible tubular sheath having proximal and distal ends and an outer
   diameter of up to about of 1000 microns and an atraumatic distal tip into the suprachoroidal space;
b) advancing the sheath to the anterior region of the suprachoroidal space; and
c) delivering energy or material from the distal end to form a space for aqueous humor drainage.

The energy may comprise mechanical, thermal, laser, or electrical energy sufficient to treat or remove scleral tissue in the vicinity of the distal end. The material may comprise a space-maintaining material.

In another embodiment, a method is provided for treating the posterior region of an eye comprising
a) inserting a flexible tubular sheath having proximal and distal ends and an outer diameter of up to about 1000 micron into the suprachoroidal space;
b) advancing the sheath to the posterior region of the suprachoroidal space; and
c) delivering energy or material from the distal end sufficient to treat the macula, retina, optic nerve or choroid.

The energy may comprise mechanical, thermal, laser, or electrical energy sufficient to treat tissues in the vicinity of the distal end. The material may comprise a drug or a drug and hyaluronic acid. The drug may comprise a neuroprotecting agent, an anti-angiogenesis agent and/or an anti-inflammatory agent. A typical anti-inflammatory agent comprises a steroid.

In another embodiment, a method is provided for treating the tissues within or adjacent to the suprachoroidal space of an eye comprising
a) inserting a composite flexible microcannula device having proximal and distal ends and an outer diameter of up to about 1000 microns into the suprachoroidal space, the device comprising an atraumatic distal tip and an optical fiber to provide detection of tissues in the vicinity of the distal tip;
b) advancing the device to the posterior region of the suprachoroidal space;
c) detecting and characterizing tissues in the suprachoroidal space to identify target tissues; and
d) delivering energy from the distal end to treat the target tissues.

The energy may comprise laser light, thermal, ultrasound or electrical energy.

Typical target tissues comprise blood vessels.

### BRIEF DESCRIPTION OF THE FIGURES

FIG. 1 is a diagram of a flexible microcannula device according to the invention.
FIG. 2 is a diagram of a microcannula device with a reinforcing member according to the invention.
FIG. 3 is a diagram of a microcannula device having a signal-emitting beacon at the distal tip according to the invention.
FIG. 4 shows of a microcannula device according to the invention positioned within the suprachoroidal space of the eye.
FIG. 5 shows a microcannula device according to the invention positioned within the suprachoroidal space and receiving a charge of drugs delivered to the posterior region of the eye through the distal end.

### DETAILED DESCRIPTION OF THE INVENTION

The present invention provides tools, materials and related methods to surgically access the suprachoroidal space of an eye for the purpose of performing minimally invasive surgery or to deliver drugs to the eye. Specifically, the invention provides a flexible microcannula device that may be placed into the suprachoroidal space through a small incision of the overlying tissues, maneuvered into the appropriate region of the space, and then activated to treat tissues adjacent to the distal tip of the device. The device may also include features for treating tissues adjacent a region along the length of the device. The treatments accomplished by the invention include mechanical modification of adjacent tissues, the delivery of energy to adjacent tissues, the delivery of drugs or drug delivery materials from the distal end of the device, or the delivery of an implant.

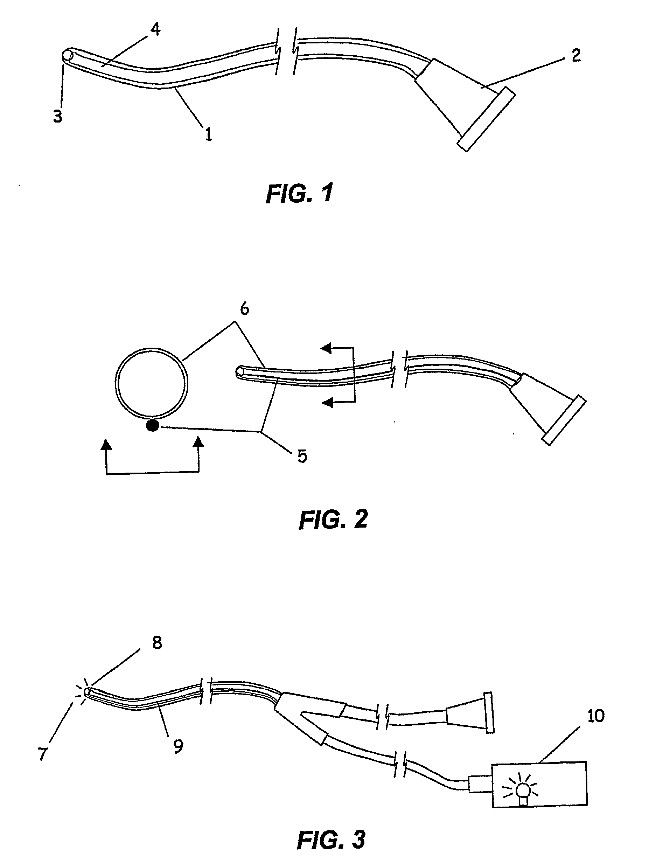

Referring to FIG. 1, a microcannula device is shown comprising a flexible elongated element 1 in the form of a tubular sheath with a connector at the proximal end 2, a distal tip 3, and a communicating channel 4. The communicating channel 4 may be used to deliver fluids, drugs, materials, energy, gases, suction, surgical tools and implants from the microcannula or the proximal connector to a distal site for a variety of tasks. The communicating channel 4 may be the lumen of a tubular elongated element to transport materials, a fiber optic to transport light energy, or a wire to transport electrical signals. A microcannula of the present invention may comprise one or more elongated elements, each having one or more communicating channels. In one embodiment, the microcannula may consist of two or more elongated elements with a reinforcing member to form a composite structure. The components may be adhered together, nested coaxially, or placed within an outer sheath, such as heat shrink tubing. One of the elements may be used for transport of materials, another for transport of light or energy, thus providing a multifunctional surgical tool.

Each elongated element may comprise a thin walled polymer or metal tube of sufficient stiffness to allow it to be advanced along the suprachoroidal space, but it should be flexible at least at its distal end. The proximal connector 2 may be of a lLuer type or similar system for the attachment or introduction of secondary elements or may be designed for attachment to specific components. To minimize the size of the suprachoroidal space occupied by the microcannula device it should be appropriately sized. The device can have an outer diameter up to about 1000 microns. Typically, the microcannula device is sized in the range of about 50 to about 1000 microns outer diameter with a wall thickness from about 10-200 microns. The cross-section of the microcannula device may be round or ovoid to approximate the shape of the suprachoroidal space.

In one embodiment, a predetermined curvature may be applied to the microcannula device to approximate the curvature of the eye, the curvature being in the range of 12 to 15 mm radius. The length of the microcannula is preferred to be long enough to reach the posterior region of the suprachoroidal space from an anterior access point, approximately 20 to 30 mm. Suitable materials for the elongated element include metals, polymers such as polyetheretherketone (PEEK), polyimide, polyamide or polyether-block co-polyamide (Pebax), polysulfone, fluoropolymers, polypropylene, polyethylene or similar materials. Preferred materials for the sheath include polyamide, polyimide, polyether block amide, polyethylene terephthalate, polypropylene, polyethylene or fluoropolymer. The microcannula device may also comprise surface treatments such as lubricious coatings or markings on the exterior for assessment of depth in the suprachoroidal space.

In one embodiment the microcannula comprises an inner member which fits and slides within the elongated element, the inner member having a proximal end and a distal tip. Advancement or withdrawal of the inner member may be used to change the shape of the distal tip of the microcannula, or alternatively to effect a mechanical action at the distal tip to manipulate tissues or deliver an implant.

The microcannula of the present invention incorporates features that enable it to be placed into and maneuvered in the suprachoroidal space. A key feature is to have the appropriate combination of axial stiffness and compliance. To achieve this, referring to FIG. 2, it may be required to use a reinforcing element 5 attached to an elongated element 6, allowing smaller overall wall thickness of the element 6 to maximize the cross-sectional dimension of the communicating channel. The reinforcing element may comprise any high modulus material such as metals including stainless steel, titanium, cobalt chrome alloys, tungsten and nickel titanium alloys, ceramic fibers and high strength polymer composites. The reinforcing element may comprise wires, coils or similar configurations. The reinforcing element or multiple elements may also be configured to provide a preferred deflection orientation of the microcannula. The reinforcing element may also be a malleable material such as a metal, to allow the surgeon to set a preferred geometry.

For optimal use in the suprachoroidal space, the microcannula is preferred to be flexible at the distal end, but transitioning to more rigid mechanical compliance toward the proximal end. The transition may comprise one or more steps in mechanical compliance, or a gradient of compliance along the length of the microcannula. It is also preferred that the distal tip of the device be atraumatic. The distal tip may incorporate a rounded shape or comprises a highly flexible material to prevent tissue damage during advancement of the device within the suprachoroidal space. The microcannula may also incorporate mechanical elements along its length to direct the shape and orientation of the distal tip, allowing the surgeon to steer the microcannula while placing it in the suprachoroidal space.

An important feature of the device is the capability of being visualized within the suprachoroidal space to allow guidance by the surgeon. The use of high resolution, non-invasive medical imaging, such as high frequency ultrasound imaging, optical coherence tomography (OCT), or indirect ophthalmoscopy, may be used in conjunction with the microcannula device of the invention. The patient eye may be imaged to determine suitable avascular sites on the overlying tissues for introduction of the device. The suprachoroidal space may also be imaged to determine the best regions for introducing or advancing the microcannula device to minimize potential trauma. The use of an ultrasound or optical contrast agent, either delivered directly to the suprachoroidal space or systemically to the subject, may facilitate imaging. Material selection and the use of contrast markers at the distal end and along the length of the microcannula device may be utilized to provide the desired imaging properties for the device and facilitate image guidance.

Visualization of the microcannula in-situ may also be accomplished by direct imaging via an endoscope placed in the suprachoroidal space. A flexible endoscope may be used to track alongside the microcannula as it advances. The endoscope should be constructed on a similar size scale to the microcannula and may be a separate device used in conjunction with the microcannula or fabricated as part of the microcannula. In one embodiment of the invention, an imaging element such as a fiber optic bundle or gradient index lens imaging rod is fabricated to be co-linear with the elongated element, creating a device with an oval cross section. Due to the shape of the suprachoroidal space, the long axis of the combined device may be significantly larger in dimension than the short axis, as long as the long axis is maintained parallel to the surface of the scleral and choroidal tissues during advancement.

A signal-emitting beacon incorporated into the microcannula enhances guidance of the device. Referring to FIG. 3, the microcannula **9** is fitted with a signaling beacon **7** to identify the location of the microcannula distal tip **8** relative to the target tissues. The signaling beacon **7** may be compatible with medical imaging techniques used to guide the surgical procedure, or it may be made for direct visualization by the surgeon. For example, the beacon **7** may comprise an echogenic material for ultrasound guidance, an optically active material for optical guidance or a light source for visual guidance.

In one embodiment, a plastic optical fiber (POF) may be incorporated to provide a bright visual light source at the distal tip **8.** The distal tip of the POF is positioned near or slightly beyond the end of the sheath of the microcannula and the emitted signal may be detected visually through either the scleral tissues on the outside of the eye or through the choroidal tissues and the pupillary aperture. Such a signaling beacon allows the distal end to be placed by the surgeon into the suprachoroidal space and advanced under visual guidance through the sclera to confirm proper introduction and placement. The microcannula may then be advanced within the suprachoroidal space to the area of desired tissue treatment under direct visualization. For treatment of posterior regions of the eye, the signaling beacon may be visualized through the papillary aperture and directed to the desired area. The POF may also comprise a tip which is beveled, mirrored or otherwise configured to provide for a directional beacon. A directional beacon may be configured in the range of about 45 to about 135 degrees from the microcannula axis to align with the direction and region of tissue treatment from the distal end of the device. The beacon may be illuminated by a light source **10,** such as a laser, laser diode, light-emitting diode, or an incandescent source such as a mercury halogen lamp. The beacon may also extend the along the length of the microcannula to indicate the orientation of the microcannula to aid surgical placement.

The microcannula device may be used to perform surgery at the distal end of the device. The distal end of the device may incorporate elements that allow for therapeutic intervention to the tissues. For example, the distal end may be advanced near the anterior region of the suprachoroidal space and the device activated to treat tissues adjacent to the distal tip. The tissue treatment may comprise the cutting or removal of tissues to form a cyclodialysis cleft, the ablation of tissues to enhance uveal scleral drainage or the placement of an implant to increase uveal scleral drainage. The distal end may also be advanced to any region of the suprachoroidal space requiring treatment of the choroids, macula, or retina. The tissue treatment may comprise the application of suction to drain suprachoroidal hemorrhage or choroidal effusion, or the treatment of the optic nerve sheath to relieve retinal vein occlusion. The tissue treatment may also comprise the application of energy or surgical tools to treat choroidal neovscularization, melanoma or nevus. Various forms of energy application may be accomplished using suitably adapted microcannulae, including laser, electrical such as radio frequency ultrasound, thermal and mechanical energy. In such a case, the device additionally comprises an inner member with a proximal end and a distal end, wherein the sheath of the microcannula and inner member are sized such that the inner member fits slidably within the sheath and the distal end of the inner member is adapted to provide tissue treatment to the eye through one or more openings in the distal end. The distal end of the inner member may be adapted for tissue dissection, cutting, ablation or removal. The inner member may be curved in the range of 12 to 15 mm radius and may comprise a multi-lumen tube and/or an optical fiber. The inner member may be made of steel, nickel titanium alloy or tungsten.

In one embodiment of the invention, the microcannula device incorporates imaging element to allow the surgeon to view, characterize, and treat blood vessels from the suprachoroidal space. For example, the device may incorporate an endoscope to image the local tissues and blood vessels. The imaging may incorporate non-visual wavelengths of light such as infra-red to aid tissue penetration. When energy is delivered by the microcannula, the area of energy delivery may be aligned to coincide with a specific area of the imaging means to facilitate specific tissue targeting by the surgeon. The imaging may also include elements to characterize blood flow, such as Doppler flow methods, to identify target vessels for treatment. The treatment method may also incorporate the use of localized labeling of target vasculature with photosensitive agents such as used in photodynamic therapy. After characterization and identification of target blood vessels, the microcannula may be used to deliver energy such as laser light or radio frequency energy to the vessels to reduce neovascularization or blood vessel leakage.

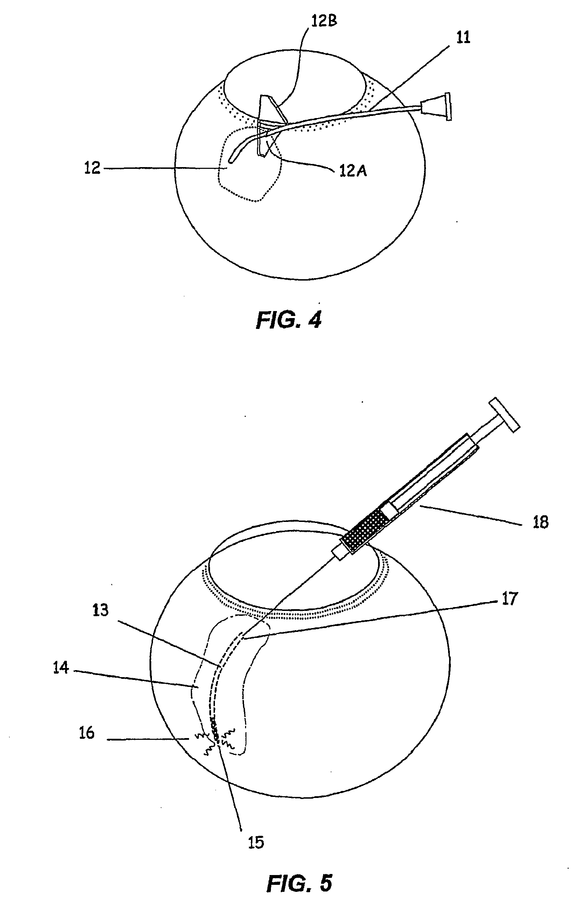

The microcannula may also be used to deliver drugs or drug delivery implants from the distal end of the device. Referring to FIG. 4, the microcannula **11** may be advanced in the suprachoroidal space to the posterior pole **12** via a surgical entry point **12A** formed by a surgical formed scleral flap **12B.** The microcannula may be used to deliver drugs or drug delivery implants to the target site. The drug or drug containing material may be delivered either from a storage space in the microcannula or by transport from a proximal connector **2** (FIG.1) through a lumen of the microcannula. Drug containing materials that provide sustained release over time are of particular utility. The materials may be delivered near the optic nerve to treat nerve damage from glaucoma, or delivered in the suprachoroidal space to treat choroidal or retinal diseases, including macular degeneration, macular edema, retinopathy, or cancer. In one embodiment, the microcannula is used to deliver microparticles of drug to the suprachoroidal space to provide a sustained release of drug to diseased tissues. The microcannula must be appropriately sized, with a lumen dimension of five to ten times the mean size of the drug microparticles, with a smooth flow path to prevent obstruction by the microparticles. The microparticles may be formulated into a suspension and injected through the microcannula at the appropriate location of the eye to provide highly localized drug concentration. A typical drug formulation may comprise drug microparticles suspended in a hyaluronic acid solution. The drug may also be delivered to the suprachoroidal space as a solid dosage form, either in the form of microparticles, a filament or a drug releasing implant designed to reside in the suprachoroidal space.

Referring to FIG. 5, a microcannula **13** is designed as a permanent implant, residing in the suprachoroidal space **14.** The distal end **15** of the microcannula is adapted to deliver drugs **16** over a sustained period to the posterior region of the eye. The distal end may incorporate microporosity or diffusional barriers to provide the appropriate drug release kinetics. The proximal end **17** of the microcannula is implanted to extend outside of the suprachoroidal space, and is positioned within the sclera or into the subconjunctival space. The proximal end **17** incorporates a self-sealing septum (not shown) that allows repeated injection into the device with a syringe **18** to refill the device with drug. The proximal end **17** may be placed in the anterior region of the eye to facilitate access. The distal end **15** may be positioned near the optic nerve or the region of retina or macula to be treated. The device may be used to provide sustained delivery of drugs such as neuroprotectants to treat damage to the optic nerve, anti-angiogenesis agents to treat macular degeneration and anti-inflammatory agents to treat inflammation in the posterior segment of the eye. The microcannula implant may also contain space-maintaining materials, such as hyaluronic acid. Also, the implant may be provided with a signal-producing beacon to locate the distal end within the suprachoroidal space during implantation. The microcannula of this embodiment is preferably constructed from materials suitable for implantation in soft tissues. Such materials include polymers such as polydimethylsiloxane, polyurethanes, Teflon, silicone-urethane copolymers, polyether-block co-polyamide, polyamide, and polyamide. The implant microcannula may also utilize secondary elements such as an outer or inner microcannula to facilitate surgical implantation. The outer surface of the implant microcannula may also incorporate features for *in situ* mechanical securement , such as tissue ingrowth porosity or features for suture anchoring.

The invention also provides methods to treat an eye by surgically accessing the suprachoroidal space. The following methods are provided as explanatory and do not constitute the entire scope of methods which may be used in conjunction with the devices described herein. In a first example, the surgeon accesses the suprachoroidal space and places a microcannula device having an atraumatic distal end within the space. A microcannula device comprising a sheath with an inner member and beacon signal is used, wherein the inner member has a distal tip configured to treat or excise tissue. The device is advanced within the space while visualizing the beacon signal to position the device tip to a location desired for surgical treatment. The device is actuated to treat a controlled amount of tissues adjacent to the distal tip. The energy may comprise mechanical, thermal, laser, or electrical energy sufficient to treat or remove scleral tissue in the vicinity of the distal end. The surgical treatment may include: formation of a space for aqueous humor drainage; treatment of the macula, retina, optic nerve or choroids in the posterior region of the suprachoroidal space; treating blood vessels within or adjacent to the suprachoroidal space. To treat blood vessels, the device preferably is adapted with an optical fiber to provide the capability of detecting and characterizing tissues and identifying target vessels before delivery of the treatment. After the surgical treatment, the device is removed and the access site is then sealed by any requisite method.

In another embodiment, the suprachoroidal space is surgically accessed and a microcannula device placed within the space. A microcannula device comprising a tubular sheath incorporating a beacon signal at the distal end is used. The device is advanced within the suprachoroidal space while visualizing the beacon signal first through the scleral tissues and second through the papillary aperture to position the device tip to a posterior location desired for drug treatment. Drugs, drug-containing materials or space-maintaining materials are delivered through the microcannula. The device is removed and the access site is then sealed by any requisite method.

The procedure may also be performed at more than site per eye as may be required. In practice, the procedure may be performed on one or more sites, and the patient monitored post-surgically. If more treatment is required, then a subsequent procedure may be performed.

The following examples are presented for the purpose of illustration and are not intended to limit the invention in any manner.

### EXAMPLE 1

A microcannula comprising a polyimide infusion lumen, a stainless steel anti-kink core wire and a plastic optical fiber to create a beacon signal at the device tip was fabricated. The components were bound together using very thin walled heat shrink tubing of polyethylene terephthalate (PET). The assembled microcannula was approximately 200 microns in outer diameter, 75 microns inner diameter and with a working length of 25mm. An atraumatic ball-shaped distal tip was produced by heating the end of the PET shrink tubing to it's melt point prior to assembly. The surface tension of the melt results in the creation of a rounded ball-shaped tip. A stainless steel wire was placed in the lumen to maintain the lumen during the melting of the tip. The proximal end consisted of an infusion tube connected to a luer fitting, and a fiber optic light pipe connected to a 25 mW laser diode illumination source. The luer fitting was attached to an injector filled with a surgical viscoelastic (Healon GV, Advanced Medical Optics, Irvine, CA).

Enucleated human eyes were prepared for surgery. Using a radial or radial plus lateral (cross) incision, the sclera was cut down to the suprachoroidal space above the medial rectus muscle attachment near the pars plana. After accessing the suprachoroidal space, the microcannula was advanced into the space while visually observing the beacon signal at the tip. The beacon tip could be observed from the outside of the eye through the overlying sclera, and also from the inside of the eye through the interposing choroidal tissues. The tip of the device could be positioned by manipulation of the proximal end while observing the beacon signal at the device distal tip. With the microcannula directed posteriorly, the device was able to be advanced adjacent to the optic nerve. Directed laterally, the device could be advanced completely around the globe, tracking a great circle route. Directed anteriorly, the device could be advanced into Schlemm's Canal and then into the anterior chamber. In a second experiment, the microcannula was placed into the suprachoroidal space under guidance with a high frequency ultrasound imaging system. The microcannula could be observed and guided within the suprachoroidal space under imaging. An injection of viscoelastic was made while observing the site with the imaging system showing a viscoelastic dissection of the space in the area of the microcannula distal tip.

### EXAMPLE 2

A drug formulation was prepared for suprachoroidal administration by injection through a microcannula of the present invention. Three milliliters of sterile triamcinolone acetonide suspension (Kenalog 40, 40 mg/ml, Bristol Meyers Squib) was withdrawn into a sterile syringe. The syringe was attached to a sterile 0.45 micron syringe filter and the drug suspension was injected into the filter, capturing the drug particles. A second syringe with an adjunct mixer was attached to the filter and 0.6 milliliters of sterile hyaluronic acid solution (Healon, 10 mg/ml, Advanced Medical Optics, Irvine, CA) introduced into the filter containing the drug particles. The hyaluronic acid and drug particles were then withdrawn into the first syringe and the filter removed. The hyaluronic acid and drug particles were mixed by multiple passage between two sterile syringes. The suspended drug formulation contained 200 mg/ml triamcinolone acetonide and 10 mg/ml hyaluronic acid. The drug formulation was then transferred to a viscoelastic injector for injection through a microcannula. The mean particle size of the triamcinolone acetonide suspended in hyaluronic acid solution was measured using a Coulter Counter instrument, demonstrating a mean particle size of approximately 4 microns.

### EXAMPLE 3

Microcannulae were fabricated, comprising a communicating element of 65 Shore D durometer Pebax tubing of 0.008" x 0.0010" diameter, containing a plastic optical fiber 0.0033" diameter and a stainless steel wire 0.001" diameter within the lumen. The plastic optical fiber was connected to a laser diode light source similar to that used in Example 1 to provide for an illuminated beacon distal tip. The steel wire was incorporated to prevent kinking of the shaft. The lumen of the tube was attached to a larger plastic tube and then to a proximal Luer connector for the attachment of a syringe or viscoelastic injector. An atraumatic distal tip was created by applying a small amount of high viscosity ultraviolet cure adhesive and allowing the surface tension to create a ball-shaped tip prior to curing. The devices were sterilized for use by gamma irradiation.

Animal studies were performed to evaluate the microcannula in accessing the suprachoroidal space and advancing to the posterior pole. The study was performed using juvenile farm pigs. In each surgery, the animals were anesthetized and prepared per standard ophthalmic surgical procedures. A limbal perotomy was performed to retract the conjunctiva. A small scleral incision was made in the pars plana region down to the choroid layer. The microcannula was inserted into the incision to access the suprachoroidal space and then advanced back to the posterior pole. Surgical microscope visualization through the pupillary aperture indicated the location of the microcannula distal tip by observing the illuminated beacon tip. The microcannula could be advanced to the posterior region of the eye without difficulty or visible tissue trauma.

### Example 4

Microcannulae similar to those used in Example 3 were made without the atraumatic tip. The devices were used during the porcine animal study as detailed in Example 3. In one case, the microcannula was unable to be advanced into the posterior region, appearing to be caught on the tissues of the suprachoroidal space. In a second case, the microcannula was able to advance to the posterior pole, but was seen to catch on the choroidal tissues in a number of locations, causing tissue irregularities visible upon angiographic imaging. In the remaining trials, the microcannulae without atraumatic tipping were able to be advanced in the suprachoroidal space. Iit was noted in each case that the devices were more difficult to advance than those with an atraumatic tip.

### Example 5

Microcannulae were fabricated and used in porcine animal studies as described in Example 3.

A viscoelastic (Healon, Advanced Medical Optics, Irvine, CA) or a steroid/viscoelastic (triamcinilone acetonide plus Healon) formulation as described in Example 2 was delivered to the suprachoroidal space in the region of the area centralis. Viscoelastic and steroid/viscoelastic delivery amounts ranged from 1.2 to 9.2 mg. The delivered materials could be observed in the suprachoroidal space by direct visualization and by posterior segment imaging using a scanning laser ophthalmoscope. Animals were survived up to one month. Posterior segment imaging at sacrifice did not show any observable changes to the retinal or choroidal blood flow, and no adverse tissue reactions were seen.

### EXAMPLE 6

A flexible microcannula comprising a small endoscope was fabricated for use in the suprachoroidal space. An experiment was performed to evaluate the use of the microcannula for direct imaging of the scleral and choroidal tissues from within the suprachoroidal space. A custom micro-endoscope (Nanoptics Inc., Gainesville, FL) consisting of about 3000 glass fibers was fabricated. The micro-endoscope had an external jacket dimension of about 250 microns terminating in a 350 micron diameter tip that included a gradient lens objective with a 5 mm focus. The micro-endoscope was coupled via a 10x Mitutoyo microscope objective and tube lens to a CCD video camera, and then to a video monitor.

An enucleated human cadaver eye was used for the experiment. A radial incision at the pars plana was made to the depth of the choroid. A small amount of viscoelastic (Healon GV, Advanced Medical Optics, Irvine, CA) was injected into the surgical incision to open the suprachoroidal space for placement of the micro-endoscope and to lubricate the passage. The micro-endoscope was inserted into the incision and advanced posterior in the suprachoroidal space. Transillumination was provided by the surgical microscope, which was adjusted to provide the best image without saturating the camera image. The micro-endoscope was advanced and manipulated to view various locations within the space. The tissues could be easily identified, the sclera appeared as a white colored bright tissue (due to the transillumination) and the choroid appeared dark reddish brown with details of the choroidal surface discernable.

### EXAMPLE 7

An indwelling microcannula implant to provide repeated access to the suprachoroidal space was fabricated. The microcannula comprised Pebax polymer tubing 0.010" ID x 0.012" OD. An atraumatic distal tip was created by applying a high viscosity ultraviolet cure adhesive to the tubing end, thus forming a rounded tip. A tissue interfacing flange was created at the proximal end by applying heat to the end of the tube, causing it to flare outwards. The total length of the microcannula was 0.79". The indwelling microcannula was placed over a delivery microcannula similar to the microcannula of Example 1 with a 4" working length. The delivery microcannula was 0.008" OD and contained a plastic optical fiber to provide for an illuminated distal tip. The proximal end of the fiber was connected to a battery powered laser diode source as described in Example 1. The delivery microcannula was sized to fit snugly inside the indwelling microcannula.

An enucleated human cadaver eye was used for the experiment. A radial incision at the pars plana was made, the incision going through the sclera and exposing the choroid. A small amount of viscoelastic fluid (Healon, Advanced Medical Optics, Irvine, CA) was injected into the suprachoroidal space at the incision in order to dissect the choroid from the sclera sufficiently to allow placement of the microcannula.

The laser diode was activated, providing a red light beacon tip on the delivery microcannula. The assembly was placed into the suprachoroidal space and advanced under visual guidance toward the posterior pole. The assembly was advanced until the tissue flange of the indwelling microcannula was flush with the scleral surface. Examination of the exterior of the eye showed the beacon tip was located near the macular region.

The delivery microcannula was withdrawn, while holding the indwelling microcannula in place with a pair of forceps. The incision was sealed with cyanoacrylate adhesive. Using a 1cc syringe, a small amount of methylene blue dye was injected into the exposed lumen of the indwelling microcannula using a 31 gauge hypodermic needle. After completion of the injection, a small incision was made through the sclera at the macular region near the distal tip of the microcannula. Methylene blue dye was seen at this incision confirming the delivery of the injection to the posterior region of the suprachoroidal space from an injection into the proximal end of the microcannula located in the anterior region.

## Claims

1. A method of detecting and characterising tissue in the suprachoroidal space in the eye to identify target tissues, the method comprising:
imaging tissue within or adjacent to the suprachoroidal space using an imaging element co-linear with a flexible microcannula device having proximal and distal ends pre-inserted into and pre-advanced to the posterior region of the suprachoroidal space.

2. The method as claimed in claim 1 wherein the imaging element comprising at least one optical fiber.

3. The method as claimed in claims 1 or 2 further comprising illuminating target tissue with light emitted from a fiber optic co-linear with the microcannula.

4. The method as claimed in claim 3 further comprising visualising the illuminated tissue within the suprachoroidal space through the choroidal tissues and the pupillary aperture.

5. The method as claimed in any preceding claim further comprising pre-delivering an optical contrast agent to the suprachoroidal space.

6. The method as claimed in any preceding claim further comprising pre-labelling target vasculature tissue with a photosensitive agent.

7. The method as claimed in any preceding claim comprising imaging the scleral and choroidal tissues.

8. The method as claimed in any preceding claim comprising imaging blood vessels within or adjacent the suprachoroidal space.

9. The method as claimed in any preceding claim comprising imaging tissues from within the suprachoroidal space.

10. A method of imaging tissues in the suprachoroidal space in the eye comprising:
imaging tissues within or adjacent to the suprachoroidal space from within the suprachoroidal space via a camera connected to a flexible microcannula having proximal and distal ends and an atraumatic distal tip, the microcannula comprising an optical fiber, and the microcannula being pre-inserted into and pre-advanced to the posterior region of the suprachoroidal space.

11. The method as claimed in claim 10 comprising transillumination of the tissues within or adjacent to the suprachoroidal space.

12. The method as claimed in claim 10 further comprising pre-delivering a viscoelastic to the suprachoroidal space prior to the pre-inserting and pre-delivering of the microcannula and endoscope into and within the suprachoroidal space.

13. A method for imaging position of an instrument in the suprachoroidal space in the eye, the method comprising and **characterised by**:
directly visually viewing from outside the eye through the choroidal tissues and the pupillary aperture, a visual light beacon emitted from a beacon tip of the instrument in the suprachoroidal space in the eye.
